# EUROPEAN PATENT SPECIFICATION

(11) **EP 2 996 777 B1**
(45) Date of publication and mention of the grant of the patent: **01.03.2017**
(21) Application number: 14727416.1
(22) Date of filing: 08.05.2014
(51) Int. Cl.: A61Q 11/00, A61K 8/73, A61K 8/02

(54) **ORAL CARE COMPOSITIONS**
MUNDPFLEGEMITTEL
COMPOSITIONS DE PROTECTION ORALE

(30) Priority: 15.05.2013 EP 13167919; 31.05.2013 EP 13169966
(43) Date of publication of application: 23.03.2016
(73) Proprietor: Unilever PLC, London, Greater London EC4Y 0DY (GB); Unilever N.V., 3013 AL Rotterdam (NL)
(72) Inventor: ANTONELLI CAMILLO, Natalie, 13271450 São Paulo (BR); COLLINS, Luisa, Zoe, Wirral Merseyside CH63 3JW (GB); MOUTINHO MONTEIRO, Karen, Cristina, 3133 AT Vlaardingen (NL)
(74) Representative: Tansley, Sally Elizabeth
(86) International application number: PCT/EP2014/059433
(87) International publication number: WO 2014/184084

(56) References cited:
- EP-A1- 1 935 395
- WO-A1-2012/087324
- WO-A2-2007/012981
- WO-A2-2012/123241
- JP-A- 2000 169 876
- JP-A- 2003 089 641
- KR-A- 20110 053 021
- US-A1- 2013 064 779

## Description

### Field of the Invention

The present invention is concerned with oral care compositions. More particularly, the present invention is concerned with mouthwash compositions

### Background of the Invention

Mouthwashes are known to remove unpleasant mouth odours and to clean teeth and gums. A further use of mouthwashes can be to whiten teeth. However there remains a need to further enhance the teeth whitening properties of mouthwashes.

Mouthwashes containing gelling agents are disclosed in KR20110053021 and WO02067883.

Teeth whitening compositions are described in EP 1 935 395.

The present invention relates to a mouthwash that enhances teeth whitening.

### Summary of the Invention

The present invention relates to an oral care mouth wash composition comprising
i) xanthan gum;
ii) gellan gum;
iii) a zinc salt and
a pigment having a hue angle, h, in the CIELAB system of from 220 to 320 degrees, and in which the composition at a temperature of 25°C has a yield stress (stress at which the shear rate is 0.1 1/s) from 0.05 to 1.0 Pa and a viscosity from 0.03 to 0.25 Pa.s (measured at a shear rate of 21 1/s).

The invention further relates to a method of instantly whitening teeth by rinsing the mouth and teeth with a composition described above.

### Detailed Description of the Invention

It is preferable if compositions of the invention are in the form of a mouthwash. The term "mouthwash" generally denotes liquid formulations which are used to rinse the surfaces of the oral cavity and provide the user with a sensation of oral cleanliness and refreshment. The mouthwash is an oral composition that is not intentionally swallowed for purposes of systemic administration of therapeutic agents, but is applied to the oral cavity, used to treat the oral cavity and then expectorated. Preferably the composition has an aqueous base.

A mouthwash composition according to the invention will usually contain an aqueous continuous phase. The amount of water generally ranges from 70 to 99% by weight based on the total weight of the mouthwash.

A mouthwash composition according to the invention will generally contain further ingredients to enhance performance and/or consumer acceptability, such as the humectants and surfactants. The amount of humectant generally ranges from 4 to 20%, more preferably from 7 to 15% by weight based on the total weight of the mouthwash. Preferred humectants that are present at the above levels include polyols, particularly preferred is sorbitol.

The viscosities and yield stress measurements are made using a thermal Analysis Discovery Hybrid Rheometer 2 (TA DHR2). The measuring geometry used on the rheometer was a 4cm diameter cone with a 2° angle. All measurements were made at 25°C.

The rheometer was used to determine the viscosity at a shear rate of 21 1/s which is taken to be the pouring shear rate. This is based on delivering a 20ml dose in 2s from a 22mm diameter bottle where the flooded depth at the exit of the neck is 10mm. Here the wall shear rate at the exit of the bottle will be of the order of 21 1/s.

In order to determine the yield stress the sample was measured using a stepped stress ramp between stresses of 0.01 Pa and 10Pa with 20 points per decade in stress. The shear rate was determined at each point over a 10s interval. The yield stress is taken as being the stress at which the shear rate is 0.1 1/s.

The composition at a temperature of 25°C has a yield stress from 0.05 to 1.0 Pa and a viscosity from 0.03 to 0.25 Pa.s, preferably at a temperature of 25°C it has a yield stress from 0.1 to 0.5 Pa and a pouring viscosity from 0.04 to 0.15 Pa.s.

The composition of the invention comprise gellan gum and xanthan gum. Preferably the weight ratio of xanthan gum to gellan gum is 1:4 to 6:1; more preferably from 1:2 to 5:1

It is preferred if the level of xanthan gum is from 0.01 to 0.5 wt% of the total composition, more preferably from 0.03 to 0.4 wt%.

It is preferred if the level of gellan gum is from 0.02 to 0.4 wt% of the total composition, more preferably from 0.03 to 0.2 wt%.

A pigment is generally understood to be a shade/material which is insoluble in the relevant medium, at the relevant temperature. This is in contrast to dyes which are soluble.

The amount of pigment in the total composition is from 0.001 to 0.1 wt%, preferably from 0.005 to 0.05 wt%.

The pigment should have a hue angle, h, in the CIELAB system of from 220 to 320 degrees most preferably between 250 and 290 degrees. A detailed description of hue angle may be found on p57 of Colour Chemistry 3rd edition by H. Zollinger published by Wiley-VCH.

Preferably, the pigment is Pigment Blue 15, more preferably Pigment Blue 15:1, 15:2, 15:3, 15:4, 15:5 or 15:6, most preferably 15:1. Particularly preferred is blue covarine. Compositions of the invention may comprise metal salt selected from citrate, lactate or chloride, preferably the metal salt is a citrate salt, more preferred citrate salts are sodium and potassium citrate; particularly preferred is potassium citrate. It is preferable if the levels of citrate salt are from 0.1 to 1 wt% of the total formulation, more preferably from 0.2 to 0.6 wt% of the total composition.

Compositions of the invention comprise a zinc salt, preferably zinc sulphate or zinc chloride, more preferably zinc sulphate heptahydrate. Preferably the level of zinc salt is from 0.05 to 1.0 wt% of the total composition more preferably from 0.1 to 0.5wt%.

It is preferable if the level of ethanol in the total composition is less than 0.1 wt%.

Compositions of the invention may comprise a preservative, a preferred preservative is sodium benzoate. Preferably the level of preservative is from 0.1 to 1 wt% of the total composition.

It is preferable if the pH of the composition at 20° C is from 4 .2 to 5.9, more preferably 4.4 to 5.3.

The composition of the invention may comprise a deposition aid. The term "deposition aid" in the context of this invention generally means a material which aids deposition of whitening agents from the composition.

Use of the composition in the context of this invention typically involves application of the composition to the oral cavity, for a recommended time, before being expectorated. The preferred time application time being from 10 to 50 seconds.

Suitable deposition aids for use in the invention will generally dissolve or disperse in water at a temperature of 25°C.

Preferred deposition aids for use in the invention are water soluble. The term "water-soluble" in this particular context generally means that the deposition aid has an aqueous solubility of at least 10g/L at 25°C, and more preferably at least 30g/L at 25°C (where the solubility is determined in un-buffered distilled water). It is particularly preferable that the deposition aid remains water soluble after drying, so that it can be re-dissolved. This prevents undesirable build up of the deposition aid on the teeth after repeated usage of the composition.

Suitable deposition aids for use in the invention include polymeric materials, preferably polymeric materials which are water soluble as defined above.

Polymeric materials for use as deposition aids in the invention may be naturally or synthetically-derived, and may be ionic or nonionic in nature.

Preferably such polymeric materials are high molecular weight. The term "high molecular weight" in this particular context generally means that the polymeric material has a molecular weight of at least 50,000, more preferably at least 500,000 g/mol. A suitable method to determine the molecular weight of such polymeric materials is gel permeation chromatography against a polyethylene glycol standard.

Specific examples of suitable classes of polymeric material for use as deposition aids in the invention include:
Water-soluble, high molecular weight linear homopolymers of ethylene oxide characterised by the general formula H(OCH₂CH₂)ₙ OH. These materials are generally termed polyethyleneoxides (or alternatively, polyoxyethylenes, or polyethylene glycols). In the general formula, n usually has an average value of at least 2000, preferably at least 50,000.

Water-soluble, high molecular weight cellulose ethers such as methylcellulose, hydroxyethylcellulose, hydroxypropylcellulose, hydroxypropyl methylcellulose, hydroxybutyl methylcellulose, hydroxyethyl ethylcellulose, sodium carboxymethylcellulose, and sodium carboxymethyl hydroxyethylcellulose.

A preferred class of polymeric material for use as deposition aids in the invention includes water-soluble, high molecular weight polymers having anionic side groups along the polymer main chain.

Specific examples of such materials include poly(carboxylic acid) polymers. Poly (carboxylic acid) polymers are typically polymers which include -COOH groups in their structure, or groups which are derived from -COOH groups such as salt, ester or anhydride groups.

For example, the poly(carboxylic acid) polymers may include:

-[C(R¹)(COOH)-]-

units in their structure, in which R¹ is selected from hydrogen, C₁₋₃ alkyl, C₁₋₃ alkoxy or C₁₋₃ hydroxyalkyl. Preferably R¹ is hydrogen.

A preferred type of poly (carboxylic acid) polymer includes adjacent:

-[C(R¹)(COOH)-]-

units in its structure (where R¹ is as defined above), for example polymers based on maleic acid, which typically include:

-{-CH(COOH)-CH(COOH)-]-

units, and/or salts or esters of such units, or such units in anhydride form in which - COOH groups on adjacent carbon atoms are cyclised to form a ring system.

For example, the poly(carboxylic acid) polymers may comprise units with pairs of carboxylic acid groups on adjacent polymer chain carbon atoms, for example polymers comprising:

-[-C(R¹)(R²)-C(R³)(R⁴)-C(R⁵)(COOH)- C(R⁶)(COOH)-]-

units in its structure (and/or salts or esters of such units, or such units in anhydride form in which -COOH groups on adjacent carbon atoms are cyclised to form a ring system); in which R¹,R²,R³,R⁴,R⁵ and R⁶ are each independently selected from hydrogen, C₁₋₃ alkyl or C₁₋₃ alkoxy. Preferably R¹ and R² are hydrogen, R³ is hydrogen and R⁴ is methoxy and R⁵ and R⁶ are hydrogen.

Such a poly(carboxylic acid) polymer may be described as the polymer based on a copolymer of methyl vinyl ether and maleic anhydride, and is commercially available for example under the trade name Gantrez®.

A particularly preferred example of such a polymer comprises:

-[-CH₂-CH(OCH₃)-CH(COOH)-CH(COOH)-]-

units in its structure, in which the -COOH groups are in free acid form. Such polymers may be linear or cross-linked. More preferably the polymer is linear. Polymers of this type are commercially available for example under the trade name Gantrez® S. Most preferably such a polymer has a molecular weight of at least 500,000 g/mol (e.g. Gantrez® S-96), ideally at least 1,000,000 g/mol (e.g. Gantrez® S-97).

Alternative polymers which may be used comprise the units as described above in anhydride form, i.e. in which the two adjacent -COOH groups are cyclised to form a ring system. Such polymers are commercially available under the tradename Gantrez® AN, e.g. Gantrez® AN-119, Gantrez® AN-903, Gantrez® AN-139 and Gantrez® AN-169.

Other alternative polymers which may be used comprise the units as described above in partial salt form, for example in which some of the free -COOH groups are converted into a metal salt of a Group I or Group II metal such as either sodium or calcium, or a mixed sodium-calcium salt. Such polymers are commercially available under the tradename Gantrez® MS, e.g. Gantrez® MS-955.

Other alternative polymers which may be used comprise the units as described above in partial ester form, for example in which some of the free -COOH groups are esterified with C₁₋₆ alkyl, e.g. ethyl or n-butyl. Such polymers are commercially available under the tradename Gantrez® ES, e.g. Gantrez® ES-225 or Gantrez® ES-425.

Mixtures of any of the above described materials may also be used.

The amount of deposition aid (as defined above) in compositions of the invention suitably ranges from 0.001 to 5.0%, preferably from 0.005 to 4.0%, more preferably from 0.01 to 2.0% by total weight deposition aid (as defined above) based on the total weight of the composition.

Although not preferred the composition may contain low levels of surfactant based on the total weight of the composition. If present, the surfactant is preferably present at levels of less than 0.5 wt% of the total composition, more preferably at levels less than 0.35 wt%, most preferably at levels of less than 0.1 wt%.

Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides.

Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines.

Mixtures of any of the above described materials may also be used.

Compositions of the present invention may also contain further optional ingredients customary in the art such as fluoride ion sources, anticalculus agents, buffers, flavouring agents, sweetening agents, colouring agents, opacifying agents, antisensitivity agents and antimicrobial agents.

The invention is further illustrated with reference to the following, non-limiting Examples. Examples of the invention are illustrated by a number, comparative examples are illustrated by a letter.

### EXAMPLE

### Formulations

Mouthwash compositions having the ingredients shown in Table 1 below were prepared.

All ingredients are expressed by weight percent of the total formulation, and as level of active ingredient.

**Table 1**

| **Ingredients** | **Wt%** | | |
|---|---|---|---|
| | **Example 1** | **Example A** | **Example B** |
| Sorbitol | 12.00 | 12.00 | 12.00 |
| Sodium Fluoride | 0.05 | 0.05 | 0.05 |
| PEG-40 Hydrogenated castor oil | 2.5 | 2.5 | 2.5 |
| Aroma | 0.5 | 0.3 | 0.5 |
| Sodium saccharin dihydrated | 0.07 | 0.1 | 0.07 |
| Surfactant (Sodium lauryl sulphate) | 0 | 0.34 | 0 |
| Zinc sulphate heptahydrate | 0.2 | 0 | 0 |
| Blue Covarine W6795 | 0.01 | 0.01 | 0.01 |
| Gantrez(S-97) | 0.5 | 0.5 | 0.5 |
| Xanthan gum | 0.05 | 0 | 0 |
| Gellan gum | 0.05 | 0 | 0 |
| Water and minors | To 100 | | |

Teeth were precleaned by brushing with a white silica paste and placed in sterile saliva for 2 hours. Baseline L*, a*, b* colour measurements were taken using a Minolta CR321 chromameter, n=10 for each group. Teeth were gently agitated in the mouthwash solution for 1 min and excess solution removed. The colour was then remeasured. Change in L*, a*, b* and WIO was calculated.

**Table 2**

| **Test Agent** | **Yield stress (Pa)** | **Pouring Viscosity (Pa.s)** | **Change in WIO (s.e.)** |
|---|---|---|---|
| Example 1 | 0.117 | 0.046 | 2.702 |
| | | | (0.379) |
| Example A | Not applicable | 0.009 | 0.243 |
| | | | (0.357) |
| Example B | Not applicable | 0.009 | 1.174 |
| | | | (0.349) |

## Claims

1. An oral care mouth wash composition comprising:
i) xanthan gum;
ii) gellan gum;
iii) a zinc salt and
iv) a pigment having a hue angle, h, in the CIELAB system of from 220 to 320 degrees, and in which the composition at a temperature of 25°C has a yield stress (stress at which the shear rate is 0.1 1/s) from 0.05 to 1.0 Pa and a viscosity from 0.03 to 0.25 Pa.s (measured at a shear rate of 21 1/s)

2. An oral care composition according to claims 1, in which the weight ratio of xanthan gum to gellan gum is from 1:2 to 5:1.

3. An oral care composition according to any preceding claim 2 in which the level of xanthan gum is from 0.01 to 0.5 wt% of the total composition.

4. An oral care composition according to any preceding claim in which the level of gellan gum is from 0.02 to 0.4 wt% of the total composition.

5. An oral care composition according to any preceding claim further comprising a metal salt selected from a citrate.

6. An oral care composition according to any preceding claim in which the zinc salt is zinc sulphate or zinc chloride.

7. An oral care composition according to any preceding claim which further comprises a deposition aid.

8. An oral care composition according to any preceding claim in which composition at a temperature of 25 °C has a yield stress from 0.1 to 0.5 Pa (stress at which the shear rate is 0.1 1/s).

9. An oral care composition according to any preceding claim in which the in which the composition at a temperature of 25 °C has a viscosity from 0.04 to 0.15 Pa.s (measured at a shear rate of 21 1/s).

10. An oral care composition according to any preceding claim in which the pigment is blue covarine.

11. An oral care composition according to any preceding claim in which the level of pigment in the total composition is from 0.001 to 0.1 wt%,

12. An oral care composition according to any preceding claim in which the pH of the composition at 20° C is from 4 .2 to 5.9.

13. An oral care composition according to any preceding claim in which the level of ethanol in the total composition is less than 0.1 wt%.

14. An oral care composition according to any preceding claim in which the level of anionic surfactant in the total composition is less than 0.35 wt%.

15. An oral care composition according to any preceding claim in which the composition is aqueous based.

16. A method of whitening teeth by rinsing the mouth and teeth with a composition described in any preceding claims.

## Patentansprüche

1. Mundpflegezusammensetzung zur Mundspülung, umfassend:
i) Xanthangummi,
ii) Gellangummi,
iii) ein Zinksalz und
iv) ein Pigment mit einem Farbwinkel h in dem CIELAB-System von 220 bis 320 Grad, und worin die Zusammensetzung bei einer Temperatur von 25°C eine Fließspannung (Spannung, bei der die Schergeschwindigkeit 0,1 1/s ist) von 0,05 bis 1,0 Pa und eine Viskosität von 0,03 bis 0,25 Pa.s (gemessen bei einer Schergeschwindigkeit von 21 1/s) aufweist.

2. Mundpflegezusammensetzung nach Anspruch 1, in der das Gewichtsverhältnis von Xanthangummi zu Gellangummi 1:2 bis 5:1 ist.

3. Mundpflegezusammensetzung nach vorhergehendem Anspruch 2, in der der Anteil an Xanthangummi 0,01 bis 0,5 Gew.-% der gesamten Zusammensetzung ist.

4. Mundpflegezusammensetzung nach irgendeinem vorhergehenden Anspruch, in der der Anteil des Gellangummis 0,02 bis 0,4 Gew.-% der gesamten Zusammensetzung ist.

5. Mundpflegezusammensetzung nach irgendeinem vorhergehenden Anspruch, die des Weiteren ein Metallsalz, ausgewählt unter einem Citrat, umfasst.

6. Mundpflegezusammensetzung nach irgendeinem vorhergehenden Anspruch, in welcher das Zinksalz Zinksulfat oder Zinkchlorid ist.

7. Mundpflegezusammensetzung nach irgendeinem vorhergehenden Anspruch, welche des Weiteren ein Ablagerungshilfsmittel umfasst.

8. Mundpflegezusammensetzung nach irgendeinem vorhergehenden Anspruch, in welcher die Zusammensetzung bei einer Temperatur von 25°C eine Fließspannung von 0,1 bis 0,5 Pa (Spannung, bei der die Schergeschwindigkeit 0,1 1/s ist) aufweist.

9. Mundpflegezusammensetzung nach irgendeinem vorhergehenden Anspruch, in der die Zusammensetzung bei einer Temperatur von 25°C eine Viskosität von 0,04 bis 0,15 Pa.s (gemessen bei einer Schergeschwindigkeit von 21 1/s) aufweist.

10. Mundpflegezusammensetzung nach irgendeinem vorhergehenden Anspruch, in welcher das Pigment blaue Kovarine darstellt.

11. Mundpflegezusammensetzung nach irgendeinem vorhergehenden Anspruch, in welcher der Anteil an Pigment in der gesamten Zusammensetzung 0,001 bis 0,1 Gew.-% ist.

12. Mundpflegezusammensetzung nach irgendeinem vorhergehenden Anspruch, in welcher der pH der Zusammensetzung bei 20°C 4,2 bis 5,9 ist.

13. Mundpflegezusammensetzung nach irgendeinem vorhergehenden Anspruch, in welcher der Anteil an Ethanol in der gesamten Zusammensetzung weniger als 0,1 Gew.-% ist.

14. Mundpflegezusammensetzung nach irgendeinem vorhergehenden Anspruch, in welcher der Anteil des anionischen Tensids in der gesamten Zusammensetzung weniger als 0,35 Gew.-% ist.

15. Mundpflegezusammensetzung nach irgendeinem vorhergehenden Anspruch, in welcher die Zusammensetzung wässrig ist.

16. Verfahren zum Aufhellen von Zähnen durch Spülen des Mundes und der Zähne mit einer in irgendeinem der vorhergehenden Ansprüche beschriebenen Zusammensetzung.

## Revendications

1. Composition de lavage de bouche pour le soin oral comprenant :
i) de la gomme xanthane ;
ii) de la gomme de gellane ;
iii) un sel de zinc et
iv) un pigment ayant un angle de teinte, h, dans le système CIELAB de 220 à 320 degrés, et dans laquelle la composition à une température de 25°C présente une limite de contrainte (contrainte à laquelle la vitesse de cisaillement est de 0,1 1/s) de 0,05 à 1,0 Pa et une viscosité de 0,03 à 0,25 Pa.s (mesurée à une vitesse de cisaillement de 21 1/s).

2. Composition pour le soin oral selon la revendication 1, dans laquelle le rapport massique de gomme xanthane à gomme de gellane est de 1:2 à 5:1.

3. Composition pour le soin oral selon la revendication 2 précédente, dans laquelle la teneur en gomme xanthane est de 0,01 à 0,5 % en masse de la composition totale.

4. Composition pour le soin oral selon l'une quelconque des revendications précédentes, dans laquelle la teneur en gomme de gellane est de 0,02 à 0,4 % en masse de la composition totale.

5. Composition pour le soin oral selon l'une quelconque des revendications précédentes comprenant de plus un sel de métal choisi parmi un citrate

6. Composition pour le soin oral selon l'une quelconque des revendications précédentes, dans laquelle le sel de zinc est le sulfate de zinc ou le chlorure de zinc.

7. Composition pour le soin oral selon l'une quelconque des revendications précédentes qui comprend de plus une aide au dépôt.

8. Composition pour le soin oral selon l'une quelconque des revendications précédentes, dans laquelle une composition à une température de 25°C présente une limite de contrainte de 0,1 à 0,5 Pa (contrainte à laquelle la vitesse de cisaillement est de 0,1 1/s).

9. Composition pour le soin oral selon l'une quelconque des revendications précédentes, dans laquelle la composition à une température de 25°C présente une viscosité de 0,04 à 0,15 Pa.s (mesurée à une vitesse de cisaillement de 211/s).

10. Composition pour le soin oral selon l'une quelconque des revendications précédentes, dans laquelle le pigment est le bleu covarine.

11. Composition pour le soin oral selon l'une quelconque des revendications précédentes, dans laquelle la teneur en pigment dans la composition totale est de 0,001 à 0,1 % en masse.

12. Composition pour le soin oral selon l'une quelconque des revendications précédentes, dans laquelle le pH de la composition à 20°C est de 4,2 à 5,9.

13. Composition pour le soin oral selon l'une quelconque des revendications précédentes, dans laquelle la teneur en éthanol dans la composition totale est inférieure à 0,1 % en masse.

14. Composition pour le soin oral selon l'une quelconque des revendications précédentes, dans laquelle la teneur en tensioactif anionique dans la composition totale est inférieure à 0,35 % en masse.

15. Composition pour le soin oral selon l'une quelconque des revendications précédentes, dans laquelle la composition est de base aqueuse.

16. Procédé de blanchiment des dents par rinçage de la bouche et des dents avec une composition décrite dans l'une quelconque des revendications précédentes.
